# EUROPEAN PATENT SPECIFICATION

(11) **EP 2 175 881 B1**
(45) Date of publication and mention of the grant of the patent: **18.07.2012**
(21) Application number: 08760823.8
(22) Date of filing: 11.06.2008
(51) Int. Cl.: A61K 39/145, A61K 9/127

(54) **INTRADERMAL INFLUENZA VACCINE**
INTRADERMALER GRIPPEIMPFSTOFF
VACCIN INTRADERMIQUE CONTRE LA GRIPPE

(30) Priority: 14.06.2007 EP 07110284; 14.06.2007 US 943967 P; 21.12.2007 US 8688 P
(43) Date of publication of application: 21.04.2010
(73) Proprietor: Crucell Switzerland AG, 3018 Bern (CH)
(72) Inventor: HERZOG, Christian, CH-4054 Basel (CH); LAZAR, Hedvika, CH-3006 Bern (CH)
(74) Representative: Verhage, Richard Abraham
(86) International application number: PCT/EP2008/057268
(87) International publication number: WO 2008/152052

(56) References cited:
- WO-A-02/07813
- WO-A-02/17985
- WO-A-02/074336
- WO-A-2004/016281
- WO-A-2004/073735
- PANCHAROEN CHITSANU ET AL: "Reduced-dose intradermal vaccination against hepatitis A with an aluminum-free vaccine is immunogenic and can lower costs." CLINICAL INFECTIOUS DISEASES : AN OFFICIAL PUBLICATION OF THE INFECTIOUS DISEASES SOCIETY OF AMERICA 15 NOV 2005, vol. 41, no. 10, 15 November 2005 (2005-11-15), pages 1537-1540, XP009092122 ISSN: 1537-6591
- HUCKRIEDE A ET AL: "The virosome concept for influenza vaccines" VACCINE, BUTTERWORTH SCIENTIFIC. GUILDFORD, GB, vol. 23, 8 July 2005 (2005-07-08), pages S26-S38, XP004986025 ISSN: 0264-410X
- KENNEY RICHARD T ET AL: "Dose sparing with intradermal injection of influenza vaccine" NEW ENGLAND JOURNAL OF MEDICINE, vol. 351, no. 22, 25 November 2004 (2004-11-25), pages 2295-2301, XP009092211 ISSN: 0028-4793
- KANRA GUELER ET AL: "Comparison of immunogenicity and tolerability of a virosome-adjuvanted and a split influenza vaccine in children" PEDIATRIC INFECTIOUS DISEASE JOURNAL, vol. 23, no. 4, April 2004 (2004-04), pages 300-306, XP009092126 ISSN: 0891-3668
- GLUECK R: "Adjuvant activity of immunopotentiating reconstituted influenza virosomes (IRIVs)" VACCINE, BUTTERWORTH SCIENTIFIC. GUILDFORD, GB, vol. 17, no. 13-14, 26 March 1999 (1999-03-26), pages 1782-1787, XP009158322 ISSN: 0264-410X

## Description

### FIELD OF THE INVENTION

The invention relates to the field of medicine and in particular to the field of infectious diseases. More in particular, the invention relates to vaccines comprising virosomes in the manufacture of medicaments for the prophylactic treatment of influenza infection.

### BACKGROUND OF THE INVENTION

Influenza viruses, members of the family of Orthomyxoviridae, are the causative agents of annual epidemics of acute respiratory disease. Influenza epidemics and pandemics continue to claim human lives and impact the global economy. In the US alone 50 million Americans get flu each year. Estimated annual deaths worldwide (1972-1992) are 60,000 (CDC statistics). Besides the seasonal epidemics, there have been three major cases of pandemic outbreaks of Influenza over the last century. The classic example of a severe influenza pandemic was the "Spanish flu" in 1918-1919 that killed an estimated 40 to 50 million people around the globe. Other pandemics occurred in 1957 (Asian flu, estimated one million deaths), and in 1968 (Hong-Kong flu, estimated 700,000 deaths). It has now been found that (lethal) avian influenza viruses can enter the human population and it has become clear that in certain cases human-to-human transmission of such avian viruses or their lethal components was indeed possible.

Infections with Influenza viruses are associated with a broad spectrum of illnesses and complications that result in substantial worldwide morbidity and mortality, especially in older people and patients with chronic illness. Vaccination against influenza is most effective in preventing the often-fatal complications associated with this infection, and much effort has been put in the development of influenza vaccines.

Three types of inactivated influenza vaccine are currently used: whole virus, split product and surface antigen or subunit vaccines. The seasonal vaccines all contain the surface glycoproteins hemagglutinin (HA) and neuraminidase (NA) proteins of the influenza virus strains that are expected to circulate in the human population in the upcoming year. The strains that deliver the HA and NA proteins incorporated in the vaccine, are grown in embryonated hens' eggs, and the viral particles are subsequently purified before further processing.

The need for the yearly adjustment of influenza vaccines is due to antigen variation caused by processes known as "antigenic drift" and "antigenic shift": Antigenic drift occurs by the accumulation of a series of point mutations in either the HA or NA protein of a virus resulting in amino acid substitutions. These substitutions prevent the binding of neutralizing antibodies, induced by previous infection, and the new variant can infect the host. Antigenic shift is the appearance of new subtypes by genetic reassortment between animal (often avian) and human Influenza A viruses. The pandemic strains of 1957 (H2N2) and 1968 (H3N2) are examples of reassorted viruses by which avian HA and or NA encoding genes were introduced in circulating human viruses, which subsequently could spread among the human population.

Based on the epidemiological surveys by over hundred National Influenza Centers worldwide, the World Health Organization (WHO) yearly recommends the composition of the influenza vaccine, usually in February for the Northern hemisphere, and in September for the Southern hemisphere. This practice limits the time window for production and standardization of the vaccine to a maximum of nine months.

In case of an urgent demand of many doses of vaccine, for example when a novel subtype of Influenza A virus arises by antigenic shift and antigenic drift, and an increased supply of vaccines is necessary, limited availability of eggs may hamper the rapid production of vaccines. Further disadvantages of this production system are the lack of flexibility, the risk of the presence of toxins and the risks of adventitious viruses, particularly retroviruses, and concerns about sterility. Some strains grow faster on eggs than others, which may hamper the speed with which such vaccines are finally delivered. Altogether, these disadvantages present a serious problem in today's practice of Influenza vaccine production using such embryonated hens' eggs. The use of a cell culture system for influenza vaccine production for epidemics as well as pandemics is therefore an attractive and reliable production alternative. The use of adenovirus-E1 transformed and immortalized cell lines for influenza virus production is disclosed in WO 01/38362.

The yearly influenza vaccine typically contains antigens from two influenza A virus strains and one influenza B strain. A standard 0.5 ml injectable dose generally contains 15 µg of hemagglutinin (HA) antigen component from each strain, adding up to approximately 45 µg HA in total, as measured by single radial immunodiffusion assay.

Due to the increasing world population, growing and emerging economies, intensified international traveling, the growing spread of yearly influenza epidemics, the threat of worldwide influenza pandemics, and the limitations of the available production facilities around the world, it is desirable to achieve protective immune responses in humans with vaccines that have improved properties as compared to the standard used at present; it is also desirable to use vaccines with lower doses (for dose sparing), whilst obtaining a similar level of protective immunity. However, when lower doses are considered, immunostimulating agents such as aluminium-based adjuvants may be considered. However, the application of aluminium in influenza vaccines is generally not applied due to the adverse side effects such as pain at the injection site.

### BRIEF DESCRIPTION OF THE FIGURES

FIG. 1 shows (A) the seroconversion (SC) rate (%), (B) the GMT-fold increase standard (>2.5 times), and (C) the seroprotection (SP) rate (>70%) in the vaccinees between 18 and 60 years of age, after intramuscular delivery (IM) or intradermal delivery (ID), for the A/New Caladenia strain (left bars), the A/Hiroshima strain (middle bars) and the B/Malaysia strain (right bars).

FIG. 2 shows (A) the seroconversion rate (%), (B) the GMT-fold increase standard (>2.5 times), and (C) the seroprotection rate (>70%) in vaccinees older than 60 years of age, after intramuscular delivery (IM) for the A/New Caladenia strain (left bars), the A/Hiroshima strain (middle bars) and the B/Malaysia strain (right bars).

FIG. 3 displays that most of the EMEA standards were met, wherein a + (plus) indicates fulfillment of the standard, and a - (minus) indicates that the standard was not met.

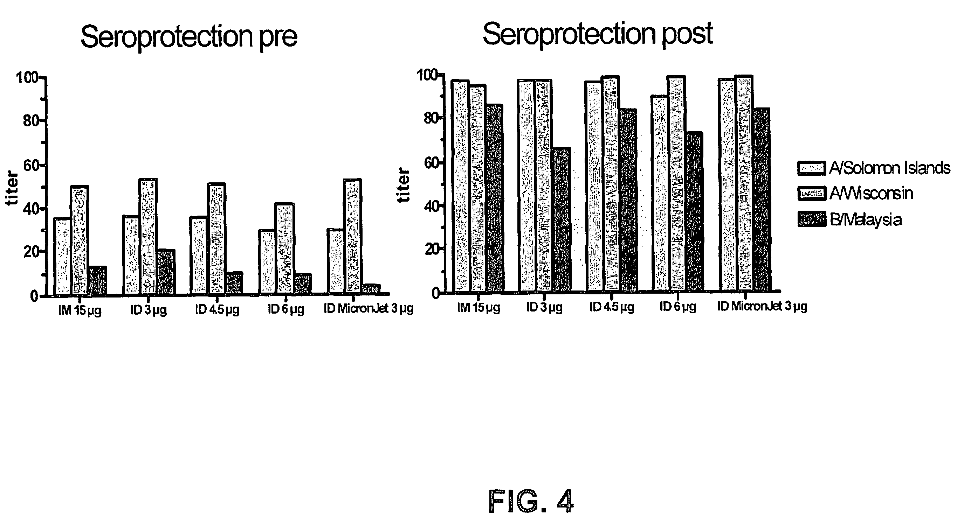

FIG. 4 shows seroconversion and seroprotection rates in a study involving the administration of 3, 4.5 and 6 µg HA antigen of three influenza strains in a single intradermal dose, compared to a high dose (15 µg HA of each strain) that is administered intramuscularly, and compared to a low dose (3 µg HA of each strain) that is administered intradermally with a device from NanoPass (generally referred to as a MicronJet device). The study was a Phase II clinical trial involving 56 human individuals in each study group, and the application of the Inflexal^{®} Influenza vaccine of the 2007/2008 flu season.

FIG. 5 shows the GMT rates pre- and post-vaccination in the groups receiving 3, 4.5 and 6 µg HA from each strain.

FIG. 6 shows the seroconversion-, seroprotection- and GMT-fold increase in the groups receiving 3, 4.5 and 6 µg HA from each strain, intradermally using the single hypodermic needle.

FIG. 7 shows the seroconversion-, seroprotection- and GMT-fold increase in the groups receiving 15 µg HA from each strain intramuscularly, and 3 µg HA from each strain intradermally using the single hypodermic needle.

FIG. 8 shows the seroconversion-, seroprotection- and GMT-fold increase in the groups receiving 15 µg HA from each strain intramuscularly, and 3 µg HA from each strain intradermally using the NanoPass MicronJet (multiple microneedle) device.

FIG. 9 shows the GMT rates pre- and post-vaccination in the groups receiving 3 µg HA from each strain, either by single hypodermic needle or using the NanoPass MicronJet (multiple microneedle) device. Each left bar represents the A/Solomon Islands strain, the middle bars represent the A/Wisconsin strain and the right bars represent the B/Malaysia strain.

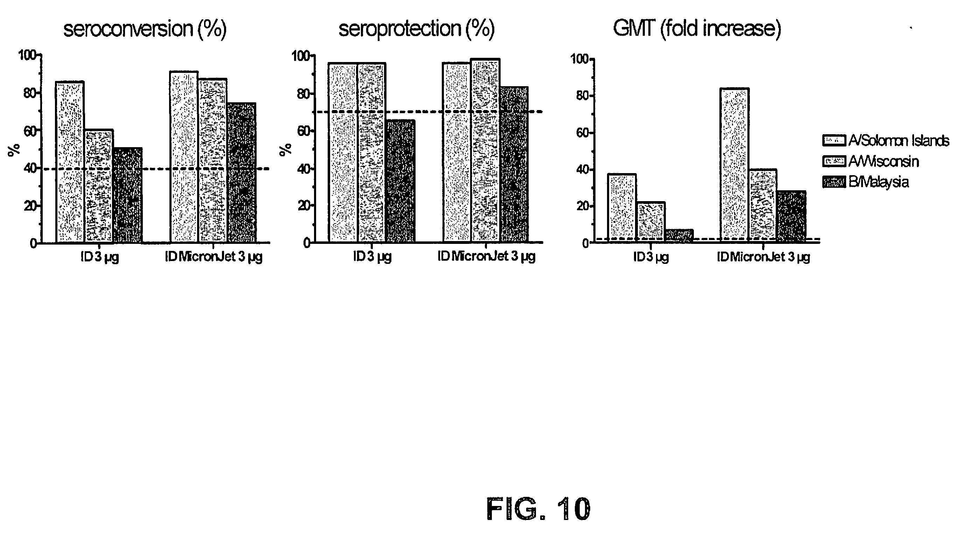

FIG. 10 shows the seroconversion-, seroprotection- and GMT-fold increase in the groups receiving 3 µg HA from each strain intradermally using the single hypodermic needle, and 3 µg HA from each strain intradermally using the NanoPass MicronJet (multiple microneedle) device.

### SUMMARY OF THE INVENTION

The present invention relates to a virosomal preparation comprising influenza hemagglutinin (HA) antigen for use as an intradermal influenza vaccine in human subjects. The art has disclosed that virosomal preparations comprising influenza HA antigen did not provide sufficient seroconversion-, seroprotection- and GMT-fold increase levels in animals (pigs). The inventors of the present invention now show that the international standards were met when these vaccines were administered to humans.

The invention further relates to a use of a virosomal preparation comprising influenza HA antigen in the manufacture of an influenza vaccine for intradermal administration in human subjects, wherein it is preferred that the preparation is an Inflexal^{®} V vaccine composition. It is furthermore preferred to manufacture such vaccines in low volumes, preferably in a single dose volume of about 0.1 mL. The invention also relates to a kit comprising a preparation according to the invention and a delivery device suitable for intradermal delivery of vaccines, preferably a multi-channel intradermal delivery device such as a MicronJet device as developed by NanoPass.

### DETAILED DESCRIPTION OF THE INVENTION

One option to improve vaccines in general is by the addition of adjuvants, or immuno-potentiating agents. A wide variety of adjuvants exist today. Typical examples are aluminium-based adjuvants, such as aluminium hydroxide or aluminium phosphate. Other examples are water-in-oil emulsions, saponins (that may come in the form of an ISCOM), and pathogen-derived toxins, such as tetanus toxoid.

Another way of stimulating immune responses towards an influenza antigen is by the generation of reconstituted influenza virosomes that essentially are reconstituted functional virus envelopes containing the hemagglutinin antigen incorporated in the envelope, but without having the genetic background of the influenza virus itself (Glück 1992; Huckriede et al. 2005). Such influenza virosomes have been approved for influenza vaccination to be used in the yearly occurring influenza epidemics, and are marketed by Berna Biotech AG under the trademark Inflexal^{®} V.

The influenza vaccines containing virosomes as described above contain typically 15 µg HA of each strain recommended by the WHO for the yearly vaccination program: two influenza A strains, and one influenza B strain. These vaccines are administered intra-muscular, generally by using an injection needle.

Besides the problems related to providing enough vaccines to meet the yearly demand for influenza vaccines (dose sparing), there are also problems in administrating these vaccines as they need to be injected with long needles: many individuals fear such needles and would benefit from other methods of administration.

Intradermal administration is a way of administering vaccines circumventing the use of long needles and the vaccines can be administered with devices that are reliable and easy to use. Moreover, skin is an excellent immune organ, because there is a high density of Langerhans cells, which are specialized dendritic cells. It is generally taken that intradermal administration of vaccines provides a more efficient uptake of antigen. There have been reports on the intradermal administration of influenza vaccines in the art (Belshe et al. 2004; Kenney et al. 2004) that showed promising results with lower dosages (3 to 6 µg HA of a single strain). Belshe et al. (2004) showed that 100% seroconversion was reached by using 6 µg HA in an intradermal administration in 119 subjects, whereas Kenney et al. (2004) showed that seroconversion and seroprotection rates were similar when an intramuscular administration of 15 µg HA was compared to a 3 µg HA administration when given intradermally in 50 subjects.

Although intradermal applications of split virus vaccines or purified antigen vaccines may be applicable, the volume, and the antigen dose are generally lower in comparison to intramuscular applied vaccine compositions, and although such studies have been performed and disclosed, no intradermal influenza vaccines are presently commercially available. To meet the standards and to be as effective as an intramuscular vaccine, it would be desirable to stimulate the immune response of such compositions (when applied intradermally) by adjuvants. However, aluminium-based adjuvants result typically in negative side effects that make it unsuitable for intradermal administration. One other way of stimulating the immune response is by using immunostimulating reconstituted influenza virosomes containing the HA antigen, wherein the antigen is delivered directly to the antigen-presenting cell through a fusion process of the virosomal membrane and the cellular membrane. Such virosome compositions are well known in the art (see, e.g., Huang et al. 1979; Kawasaki et al. 1983; Hosaka et al. 1983) whereas they have been described for the use in other types of vaccines in WO 92/19267.

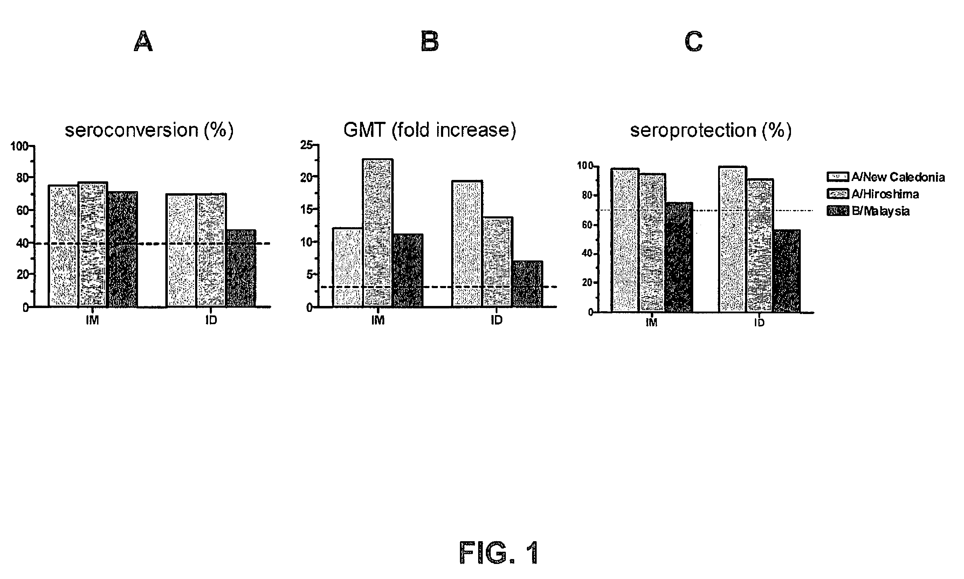

Influenza virosomes have been used for intradermal administration and such methods were disclosed in WO 2004/016281. The examples and drawings in WO 2004/016281 disclose that a virosomal-based influenza vaccine (Inflexal^{®} V) in combination with different concentrations of an ADP-ribosylating toxin (*E. coli* heat-labile enterotoxin LT) fulfills one of the criteria set by the EMEA (at least 40% seroconversion rate = the percentage of vaccinees who have at least a four-fold increase in serum hemagglutinin (HI) titers after vaccination) for each vaccine strain, see FIGS. 1 through 3 therein. However, no positive results were obtained when the adjuvant (the ADP-ribosylating toxin) was omitted, and only 3 µg HA of each strain was used. Seroconversion rates did not reach 40% in any of the cases that the virosomes without LT were administered intradermally. The ordinary way of administration, intramuscularly, did result in sufficient seroconversion. Hence, the conclusion from WO 2004/016281 would be that intradermal administration of virosome-based influenza vaccines should not be used to obtain sufficient protection.

The inventors of the present invention have now found that influenza vaccines based on virosomes, such as those marketed under the name Inflexal^{®} V can be administered intradermally (instead of being injected intramuscularly) and do result in reaching the standards as set by the authorities, when used in humans. The intradermal application according to the present invention is performed with a lower dose (and volume) than the generally required 15 µg HA of each strain, whilst maintaining to induce the protective immunity as required by the general criteria set for such vaccines (such as those set by the EMEA). Lowering the dose provides a solution for the problems with production capacities worldwide, and for the increasing demand for influenza vaccines. Intradermal administration provides also a solution for the cumbersome intramuscular injections with needles that many people fear. Moreover, by using a virosome-based vaccine a lower rate of adverse events is to be expected due to the high purity of virosomal adjuvated influenza vaccines.

Clearly, the fact that the inventors of the present invention now found that vaccines with lowered doses and in the form of a virosome, do provide sufficient protection when applied intradermally was highly unexpected in view of WO 2004/016281. The difference may be explained by the fact that the experiments in WO 2004/016281 were performed in animals, in pigs to be precise, not in humans. The inventors of the present invention have now found that intradermal administration of virosome-based influenza vaccines does provide sufficient seroconversion and seroprotection rates when low doses of HA antigen are administered to human subjects.

The present invention relates to a composition comprising about 3 µg HA antigen from a single influenza virus strain, wherein the composition may further comprise HA antigens from multiple influenza virus strains, preferably from A-type as well as B-type strains, for use in human therapy or -prophylaxis. It is to be understood that the composition may comprise HA antigens from multiple influenza strains wherein the amount of HA antigen is preferably about 3 µg per strain.

"Intradermal delivery" or "intradermal administration" as used herein means delivery of the influenza vaccine to the regions of the dermis of the skin, although it will not necessarily be located exclusively in the dermis, which is the layer in the skin located between about 1.0 and about 2.0 mm from the surface in human skin. There may be a certain amount of variation between individuals and in different parts of the body. Generally, the dermis is reached by going approximately 1.5 mm below the surface of the skin, between the stratum corneum and the epidermis at the surface and the subcutaneous layer below respectively. After administration, the vaccine may be located exclusively in the dermis or it may also be present in the epidermis.

Suitable devices for use with the intradermal vaccines include applicators such as those marketed by NanoPass Technologies Ltd. and as those described in US 4886499, US 5190521, US 5328483, US 5527288, US 4270537, US 5015235, US 5141496, US 5417662, WO 99/34850, EP 1092444, US 5480381, US 5599302, US 5334144, US 5993412, US 5649912, US 5569189, US 5704911, US 5383851, US 5893397, US 5466220, US 5339163, US 5312335, US 5503627, US 5064413, US 5520639, US 4596556, US 4790824, US 4941880, US 4940460, US 6494865, US 6569123, US 6569143, US 6689118, US 6776776, US 6808506, US 6843781, US 6986760, US 7083592, US 7083599, WO 2004/069302, WO 2004/098676, WO 2004/110535, WO 2005/018703, WO 2005/018704, WO 2005/018705, WO 2005/086773, WO 2005/115360, WO 02/02178, WO 02/02179, WO 02/083205, WO 02/083232, WO 03/066126, WO 03/094995, WO 2004/032990, WO 2004/069301, WO 97/37705, and WO 97/13537.

Any intradermal delivery device that is found suitable by the skilled person for delivery of influenza vaccines may be used according to the present invention, and may be part of a kit according to the present invention. Preferred are devices such as those developed by NanoPass and generally referred to as MicronJet devices that comprise multiple delivery channels, also referred to as microneedles or MicroPyramids. The MicronJet device used intra (in Example 2 hereinbelow) contained four separate needles, but MicronJet devices may contain other numbers of separate microneedles, such as at least two and up to a number that still allows the flow of the composition that needs to be delivered to the human skin.

Standards are applied internationally to measure the efficacy of influenza vaccines. The EMEA criteria for an effective vaccine against influenza are:

| | |
|---|---|
| Seroconversion rate: | >40% (18 to 60 years) |
| | >30% (>60 years) |
| Conversion factor: | >2.5 (18 to 60 years) |
| | >2.0 (>60 years) |
| Protection rate: | >70% (18 to 60 years) |
| | >60% (>60 years) |

Seroconversion is defined as the percentage of vaccinees who have at least a four-fold increase in serum hemagglutinin inhibition (HI) titers after vaccination, for each vaccine strain. The Conversion factor is defined as the fold increase in serum HI geometric mean titers (GMT) after vaccination, for each vaccine strain. The protection rate is defined as the percentage of vaccines with a serum HI titer equal to or greater than 1:40 after vaccination (for each vaccine strain) and is normally accepted as indicating protection.

Theoretically, to meet the European requirements, an influenza vaccine has to meet only one of the criteria outlined above, for all strains of influenza included in the vaccine. However in practice, at least two will need to be met for all strains and may be sufficient. Some strains are more immunogenic than others and may not reach the standards, even when administered intramuscularly. Often, when the standards are met for healthy individuals between 18 to 60 years, the standards may not be met in the elderly (>60 years).

The present invention relates to the use of a virosomal-based influenza vaccine preparation in the manufacture of an influenza vaccine for intradermal administration in humans. Moreover, a lower volume is administered when the vaccine is delivered intradermally.

In certain embodiments, the quantity of antigen in the composition according to the invention is between 1 and 10 µg HA of each influenza strain in a single vaccine dose, e.g. between 2 and 10 µg, e.g. about 3, 4, 5, 6, 7, 8 or 9 µg HA of each influenza strain in a single vaccine dose. The quantity of antigen in the composition according to the present invention is preferably about 3.0 µg HA of each influenza strain in a single vaccine dose; and this requires administration of only 20% of the typical intramuscular dose (0.1 mL instead of 0.5 mL).

The present invention relates to a virosomal preparation comprising influenza hemagglutinin (HA) antigen, for use as an intradermal influenza vaccine in human subjects. Preferably, the preparation does not comprise antigens from viruses other than influenza virus. Virosomes comprising HA antigens have been made for decades and methods for producing such virosomes are well known to the person skilled in the art. Virosomal preparations comprising HA antigens from influenza viruses have also been applied for other prophylactic treatments such as those manufactured for hepatitis A vaccines (Epaxal^{®}). However, in a preferred embodiment, when the treated human subject is to be vaccinated to prevent influenza infections, the preparation does not comprise antigens from other pathogens such as viruses like hepatitis A virus. The virosomal preparation comprising HA antigens from influenza virus may be used when a pandemic occurs. In that case, HA antigen from a single influenza strain is used to be part of the virosomal preparation. However, when a seasonal vaccine is being produced, such vaccines typically are trivalent in the sense that HA is present from three different influenza strains, generally two A strains and one B strain. For the treatment of human subjects during a seasonal flu campaign, it is preferred that the preparation according to the present invention comprises HA antigen from two or more influenza virus strains, preferably from three strains, more preferably from two A-type strains and one B-type strain. The preparation according to the present invention is preferably useful in the prophylactic treatment of humans and therefore preferably further comprises a pharmaceutically acceptable excipient and/or a solvent. Pharmaceutically acceptable excipients are well known in the art and may be selected for instance from lecithinum, Na₂HPO₄, KH₂PO₄ and NaCl. A suitable solvent is water.

In a preferred embodiment, the invention relates to a preparation according to the invention, wherein the concentration HA antigen from each influenza strain is about 3.0 µg per influenza strain. Generally, intramuscular administration of virosomal preparations for the seasonal flu campaign is performed with about 15 µg HA per influenza virus strain. As disclosed herein, one-fifth (20%) of such preparations used for intramuscular administration can now be applied intradermally while obtaining appropriate levels of seroconversion-, seroprotection- and GMT-fold increase. Hence, for intradermal administration, the preferred concentration of HA per influenza strain is about one-fifth: about 3.0 µg.

Preparations comprising virosomes based on influenza viruses and comprising HA antigen as the immunogenic determinant are commercially available. Sampling one-fifth of such preparations for intradermal use is preferred because of ease of production. In a preferred embodiment, the invention relates to a preparation according to the invention, wherein the preparation is an Inflexal^{®} V vaccine composition, as marketed by Berna Biotech AG, Switzerland. This vaccine typically has an HA content from different influenza strains each year, depending on the recommendations of the WHO. Preferably, the preparation according to the invention does not contain a trivalent combination of HA antigens from influenza strains A/New Caledonia/20/99, A/Moscow/10/99 and B/Hong Kong/330/2001. Other combinations of any of these strains or viruses like these strains are nevertheless possible and can be included in a preparation according to the present invention.

The present invention also relates to a use of a virosomal preparation comprising influenza HA antigen in the manufacture of an influenza vaccine for intradermal administration in human subjects. Preferably, the preparation in a use according to the invention does not comprise antigens from viruses other than influenza virus. For the seasonal application it is preferred that the preparation comprises HA antigen from two or more influenza virus strains. For a pandemic it is preferred that the single influenza strain causing the pandemic is represented by its HA antigen in a preparation used according to the present invention. In a preferred use the concentration HA antigen from each influenza strain, even when only one influenza strain is present in the preparation, is about 3.0 µg per influenza strain. Preferably, an Inflexal^{®} V vaccine composition is used for the manufacture of a medicament according to the present invention, wherein it is preferred that the preparation does not contain a trivalent combination of HA antigens from influenza strains A/New Caledonia/20/99, A/Moscow/10/99 and B/Hong Kong/330/2001.

Intradermal delivery allows for lower volumes. When the original intramuscular vaccine has a volume of 0.5 mL (such as Inflexal^{®} V in a single dose), it is preferred to use 20% of that volume in intradermal administration. Therefore, it is preferred that the vaccine is manufactured in a single dose volume of about 0.1 mL.

Virosomes may have adjuvanting activity, and could thus be considered adjuvants. It is shown herein that further adjuvants (i.e. besides virosomes) are not required when a virosomal preparation according to the invention is used for intradermal vaccination of humans against influenza. Hence, the vaccine composition of the present invention does not comprise additional adjuvants. Such additional adjuvants might have given rise to side-effects, which are thus circumvented according to the invention. Further the invention does not require the manufacture and testing of these additional adjuvant components, thus circumventing additional costs and complexity that would be associated with additional adjuvants.

The invention also relates to combinations of the preparation according to the invention and the delivery device with which it is being delivered intradermally. Hence, the invention also relates to a kit comprising a preparation according to the invention and a delivery device suitable for intradermal delivery of vaccines. Even more preferred are kits in which the preparation is already present inside the delivery device, which enables a health worker to easily administer the vaccine to the human subject. Preferred delivery devices that are used in a kit according to the invention are those devices marketed under the name MicronJet by NanoPass^{®}. These and other delivery devices that may be used in the kit according to the present invention are those disclosed in any one of the following patents and patent applications: US 4886499, US 5190521, US 5328483, US 5527288, US 4270537, US 5015235, US 5141496; US 5417662, WO 99/34850, EP 1092444, US 5480381, US 5599302, US 5334144, US 5993412, US 5649912, US 5569189, US 5704911, US 5383851, US 5893397, US 5466220, US 5339163, US 5312335, US 5503627, US 5064413, US 5520639, US 4596556, US 4790824, US 4941880, US 4940460, US 6494865, US 6569123, US 6569143, US 6689118, US 6776776, US 6808506, US 6843781, US 6986760, US 7083592, US 7083599, WO 2004/069302, WO 2004/098676, WO 2004/110535, WO 2005/018703, WO 2005/018704, WO 2005/018705, WO 2005/086773, WO 2005/115360, WO 02/02178, WO 02/02179, WO 02/083205, WO 02/083232, WO 03/066126, WO 03/094995, WO 2004/032990, WO 2004/069301, WO 97/37705, and WO 97/13537.

The invention further relates to a method of vaccinating a human subject against influenza infections, said method comprising administering intradermally to the human subject a virosomal preparation comprising influenza hemagglutinin (HA) without additional adjuvant. The invention also provides a method of vaccinating mammalian subjects against influenza infections, the method comprising the steps of preparing a virosome-based influenza vaccine comprising HA antigen an influenza strain, and administering the vaccine intradermally. Preferably, the mammals are humans. In a pandemic threat situation, the vaccine preferably comprises HA antigen only from the strain that is of interest and that causes the pandemic threat. However, during general seasonal vaccine set-ups, protocols and campaigns, it is preferred that the vaccine that is administered intradermally according to the invention is a trivalent vaccine, comprising HA from three different influenza strains, more preferably from two A-type strains and one B-type strain.

In another preferred embodiment, the vaccine comprises 1-10, e.g. about 3.0 µg HA antigen from the single strain, or in the case of the trivalent vaccine, from each of the three influenza strains. In an even more preferred embodiment, the virosome-based influenza vaccine is a vaccine commercialized by Berna Biotech AG under the trademark Inflexal^{®} V vaccine. Preferably, the invention relates to a method according to the invention wherein the vaccine does not contain a trivalent combination of HA antigens from influenza strains A/New Caledonia/20/99, A/Moscow/10/99 and B/Hong Kong/330/2001. As shown intra, administration is preferably performed by using a delivery device suitable for intradermal delivery of vaccines. A device that is suitable for intradermal delivery may be a single hypodermic needle. It is found that certain devices are made such that their needles cannot go beyond the skin layers that would result in sub-optimal intradermal deliveries. In other words, certain devices contain needles that are so short that most, if not all of the vaccine preparation is delivered intradermally. Preferred devices that are used in the methods according to the invention are delivery devices that contain two or more separate delivery channels, such as microneedles or MicroPyramids. More preferably, such a delivery device contains four or more separate delivery channels. Highly preferred is a NanoPass^{®} delivery device, such as a MicronJet device.

### EXAMPLES

### Example 1. Clinical trial with virosome-based influenza vaccine in human subjects (3 µg HA of each strain)

A clinical trial with human subjects was performed with Inflexal^{®} V to evaluate the safety and the humoral responses of an intradermally administered vaccine in a nested study group. The study was open and non-randomized. The vaccine that was used was the trivalent virosomal adjuvanted influenza vaccine Inflexal^{®} V vaccine that was being developed and studied for the 2006-2007 flu season. One dose of this intramuscular vaccine contained (originally) 15 µg hemagglutinin of each of the three following influenza strains: A/New Caledonia/20/99 (H1N1; IVR-116), A/Hiroshima/52/2005 (H3N2; IVR-142; an A/Wisconsin/67/2005 like virus) and B/Malaysia/2506/2004 coupled to virosomes in 0.5 mL solvent. The intradermal administration was performed with a 20% part of the vaccine: a single dose of 0.1 mL containing 3 µg HA of each influenza strain, using a normal injection syringe with needle. The intradermal study involved 23 healthy volunteers that were all between 18 to 60 years of age (age range: 19.2 to 59.6). The standards used were as set by the EMEA. The study was compared to an intramuscular administration that was performed with a single dose of 0.5 mL vaccine (the normal IM dose) in 56 adults that were between 18 and 60 years (age range 21.1 to 59.8) and in 58 adults that were over 60 years of age (age range 60.4 to 83.3). Sampling was performed 20 to 24 days after vaccination.

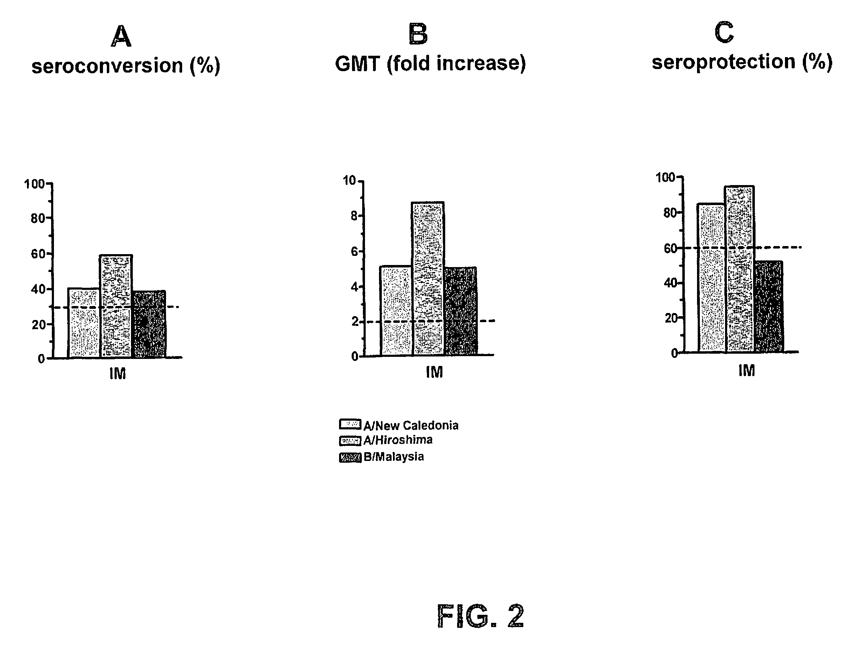

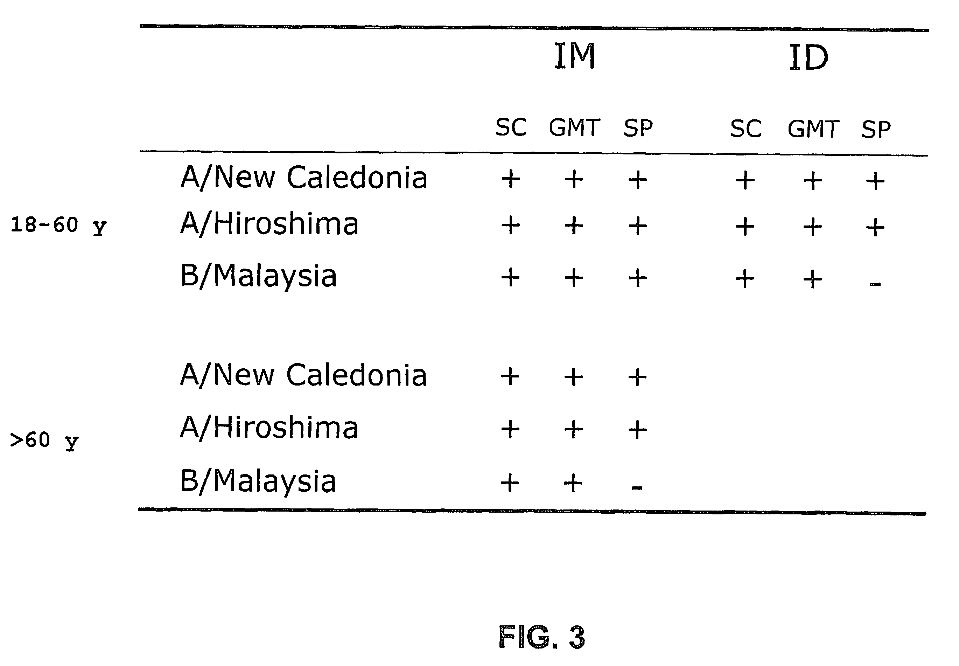

FIG. 1A shows the seroconversion rate (%) in the vaccinees after intramuscular delivery (IM) and after intradermal delivery (ID), and shows that for all three strains the set standard (>40% seroconversion) was met in both delivery methods. FIG. 1B shows that also the GMT-fold increase standard (>2.5 times) was also met for all three strains. FIG. 1C shows that seroprotection was sufficient (>70%) for the two A strains, whereas the seroprotection rate for the B strain was not met after intradermal delivery. However, when compared to the results obtained with the elderly (>60 years), as shown in FIG. 2C, also the intramuscular delivery did not result in a protection rate that was over 60%, which is the standard for this age group. This result is most likely due to the immunogenicity of the HA antigen of this B type influenza strain. FIGS. 2A and 2B show the results after intramuscular administration in the >60 years age group, indicating that levels were reached to a sufficient level to meet the EMEA standards. FIG. 3 provides an overview of the results, wherein a + (plus) indicates fulfillment of the standard, and a - (minus) indicates that the standard was not met.

These studies show that when a virosome-based influenza vaccine is administered intradermally in a concentration of 3 µg of each strain (two A-type strains, and one B-type strain) in humans, seroconversion and GMT standards are met for all three strains, whereas the seroprotection rate is met for at least the two A strains. This was highly unexpected and is in strong contrast to the findings as disclosed in WO 2004/016281, where it was shown that such vaccination regimens did not meet the standards set by the authorities, for any of the three strains used therein.

### Example 2. Dose escalation (and an intramuscular vs. intradermal) study with virosome-based influenza vaccines in human subjects

A second clinical study involving human individuals was performed to evaluate the humoral immune response of an intradermally administered seasonal virosomal adjuvanted influenza vaccine. This involved a single-center, randomized, dose escalation study wherein the trivalent Inflexal^{®} V influenza vaccine for the 2007/2008 flu season was administered intradermally in a volume of 0.1 mL, and wherein a dose comprised 3, 4.5 or 6 µg HA of each strain (A/Solomon Islands/3/2006 [H1N1]; A/Wisconsin/67/2005 [H3N2]; B/Malaysia/2506/2004). The intramuscularly delivered vaccine was taken as a positive control (containing 3 x 15 µg HA per strain in a 0.5 mL dose). Furthermore, it was tested whether a microneedle device developed by NanoPass (herein generally referred to as a MicronJet device) could also be used to deliver the antigen intradermally, and whether beneficial results could be obtained.

The Micronjet device generally makes use of multiple injection "needles" or channels with a steady and determined injection depth, in contrast to a single hypodermic needle that has only one channel and that has a higher variable injection depth (largely depending on the person administering the vaccine). The MicronJet device in general is a needle-substitute designed for painless intradermal delivery of drugs and vaccines. Mounted on a standard syringe instead of a conventional needle, the MicronJet can be used to inject virtually any substance allowing controlled intradermal delivery. It is very suitable for intradermal administration of drugs, proteins and vaccines, and requires minimal performer expertise. The head of the device contains an array of small needles, microneedles, also referred to as "MicroPyramids," each less than one-half of a millimeter high. Since the microneedles are so short, they do not reach the free nerve endings of the skin, which are responsible for pain sensation, so there is no painful "needle prick," and most substances can be administered completely without pain. The microneedles are so small, that they are barely visible to the naked eye, making a MicronJet device far less intimidating than a conventional needle, and perfect for children and needle-phobic patients.

In line with the lowest dose in the dose escalation study, the MicronJet device study group also received 3.0 µg HA of each strain. The entire study involved five groups in total. Each group contained 56 individuals, wherein three groups received an intradermal administration using a single hypodermic needle (groups A1, A2, A3), one group receiving the intramuscular injection (group B; 15 µg dose), and one group receiving the intradermal injection with a microneedle device (group C; 3.0 µg dose). Group A1 received 3 µg HA from each strain, group A2 received 4.5 µg HA from each strain, and group A3 received 6 µg HA from each strain.

On day 1, before vaccination, a pre-vaccination sample was taken, and 22 days post-immunization, another sample was obtained from each individual. Table I provides the details of the study indicating the average age within each group, the gender of the people per study group, and the GMT pre-test titer for the different strains. The parameters, GMT-fold increase, seroprotection and seroconversion were determined as in the previous example.

FIG. 4 shows the seroprotection rate of the pre-immmunization samples (left panel) as compared to the samples on day 22 after immunization (right panel) for all three strains and for all groups. It clearly shows that before vaccination, none of the groups in general contained sufficient protective titers against any of the strains, whereas each group, after receiving the vaccines, reached average seroprotection levels that in almost all cases went above the 70% threshold.

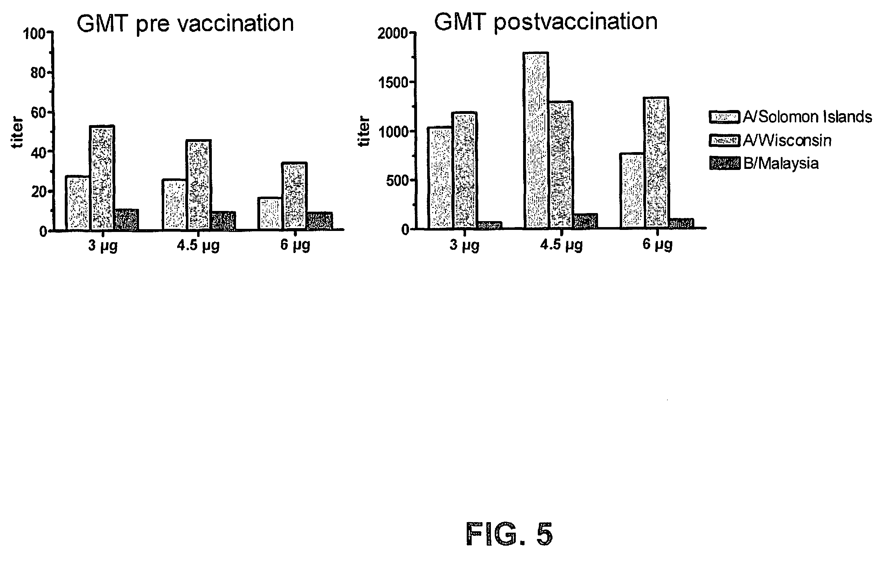

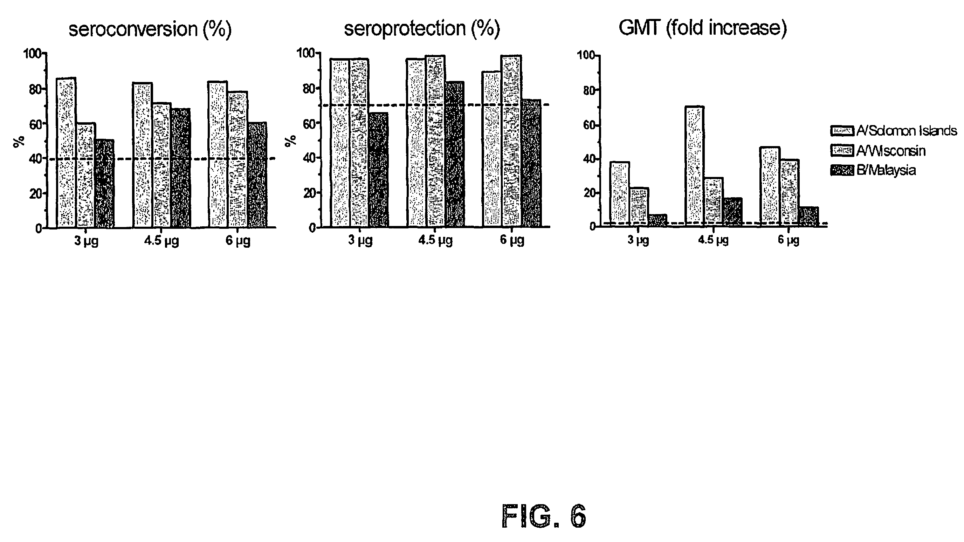

In FIG. 5, the GMT levels are depicted for the intradermal groups A1, A2, and A3, receiving the 3, 4.5 and 6 µg HA per strain via the hypodermic needle. It clearly shows that there is a substantial increase in GMT. The GMT-fold increase is shown in FIG. 6 (right panel), which indicates that the threshold (>2.5-fold) in increase (dotted line) is reached, also in the case of the B-type virus. This level is even achieved with the lowest dose of 3 µg HA antigen. FIG. 6, left and middle panel also show the seroconversion and seroprotection rates of the groups receiving these three low doses via the hypodermic needle. Except for the 3 µg HA dose of the B-strain, all vaccines provided a sufficient rate above the threshold as discussed herein (40% seroconversion and 70% seroprotection). Immunogenicity of the vaccines is thus confirmed for each strain.

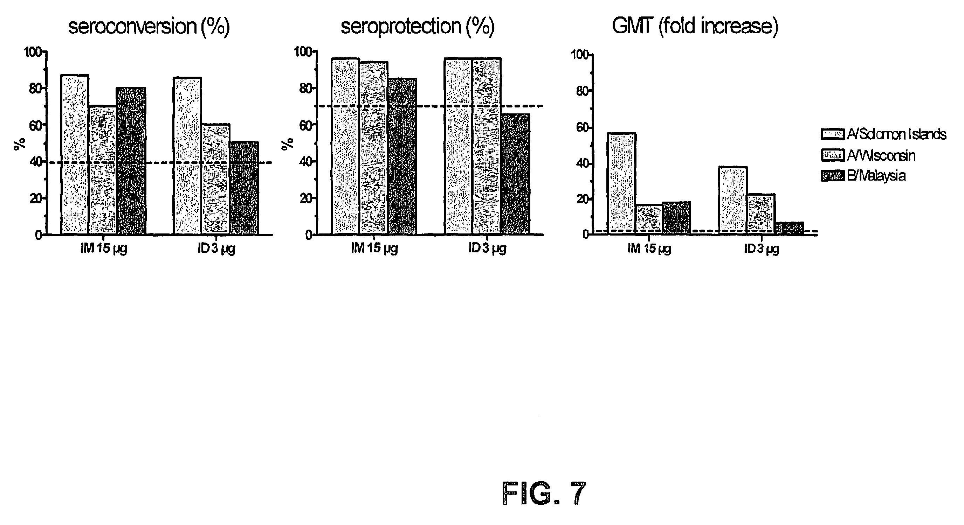

The intramuscular administration of the basic 15 µg HA dose (group B) was also compared with the 3 µg HA dose administered via the hypodermic needle (group A1). FIG. 7 shows (left panel) that sufficient seroconversion rates were obtained, while (middle panel), in line with what is shown in FIG. 6 (middle panel, left three bars), seroprotection was obtained only with respect to the A-strains, and that the seroprotection level of the B strain was just below the 70% threshold. GMT-fold increase (right panel) was sufficient in both high and lower doses administered via the intramuscular and intradermal route respectively.

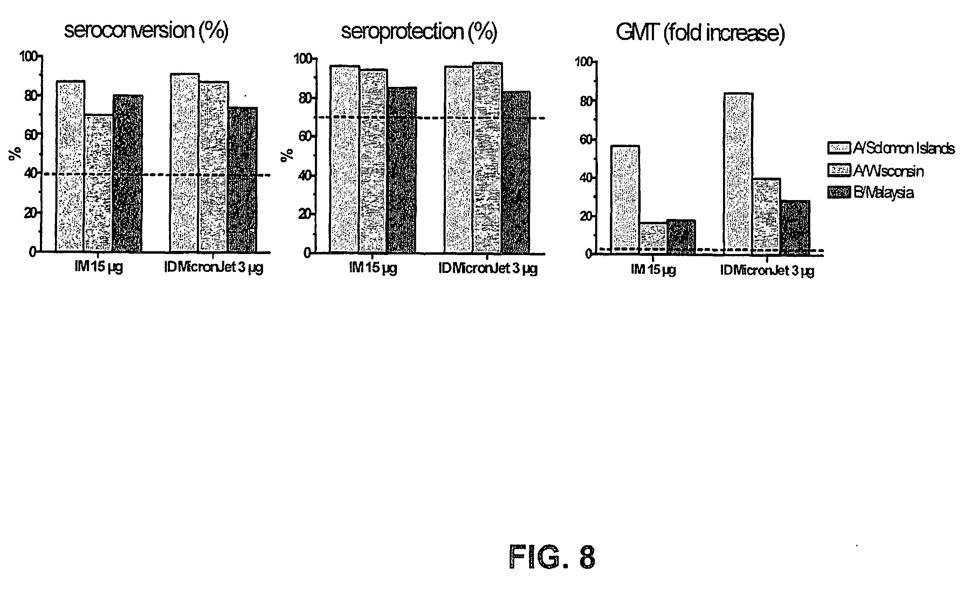

The intramuscular delivery of 15 µg HA per strain (group B) was also compared to the intradermal delivery of 3 µg HA per strain administered with the NanoPass (MicronJet) device (group C). Seroconversion rates from the low dose reached more than acceptable levels in comparison to the intramuscular high dose delivery, and importantly, also seroprotection rates (including that of the B-type virus) reached the threshold level (FIG. 8, left and middle panels). Thus, in contrast to the intradermal delivery with the single hypodermic needle, the multiple needle MicronJet device contributed extra, in that the B-type virus induced seroprotection rate was even further increased. Since the NanaoPass delivery device makes use of several very small needles (or MicroPyramids), it is concluded that using multiple injection sites at a very small piece of skin broadens the area in which the vaccine is delivered. This makes that the vaccination effect is further increased. Therefore, it is preferred to have multiple simultaneous injection sites when applying intradermal delivery. Simultaneous in this context means that at the same time, the vaccine dose is delivered to the host through multiple, separated channels in a single device, and preferably in a single shot. Preferably, more than one channel (small needle, microneedle, or MicroPyramid) is used in such a single delivery device: it is preferred to use at least two, three or four channels, and even more preferably, more than four channels are used. Most preferably, a device is used in which a high number of channels is used that still allows the flow of the vaccine composition and that allows the separation over multiple channels without clogging, and that allows the delivery of the vaccine composition that results in vaccination with sufficient (threshold-reaching) efficacy; that is, reaching acceptable seroconversion-, seroprotection-and GMT-increase levels.

FIG. 8, right panel, shows the increase in GMT titers when the pre-vaccination titers are compared to the post-vaccination titers, between the intramuscular delivery using a conventional needle and the intradermal delivery using the NanoPass device. Strikingly, when a 5x lower dose of HA antigen is administered via the intradermal route, a higher GMT titer increase is detected for each of the three strains when compared to the intramuscular route and the higher dose. It is therefore concluded that acceptable and sufficient GMT titers are obtained for A- and B-type Influenza strains and that at least a 5x lower dose (in this case from 15 µg HA dose [per strain] to 3 µg HA dose [per strain]) can be used, when a virosome-based influenza vaccine is combined with an intradermal delivery route, preferably by using a NanoPass device as disclosed.herein. This means that by using a kit according to the invention, wherein a virosome based influenza vaccine is combined with an intradermal delivery device, preferably a multichannel device such as those developed by NanoPass, a dramatic dose sparing can be achieved, resulting in a much higher number of available doses for the entire world population, especially in cases such as pandemic threats.

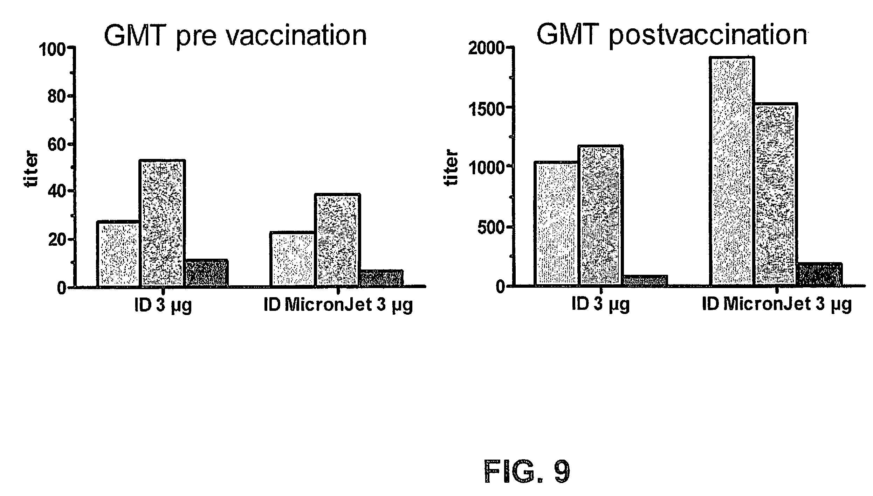

FIG. 9 shows similar results when the intradermal delivery by using one hypodermic needle (left three bars in each panel) is compared to the Micronjet device (right three bars in each panel): the spreading of the injection sites (by using multiple channels simultaneously) results in a higher increase of GMT titers. Each left bar represents the A/Solomon Islands strain, the middle bars represent the A/Wisconsin strain and the right bars represent the B/Malaysia strain. When comparing the low doses of 3 µg HA per strain either administered with a single hypodermic needle or administered with the MicronJet device, it turns out that the seroprotection rate of the B-type virus that remained below the 70% threshold level when using the single needle reaches a level above 70% when the virosome-based influenza vaccine is administered through multiple needles in the MicronJet NanoPass device. This clearly indicates the beneficial immunogenicity effect and added value of using multiple injection sites in a single shot.

After determination of the HAI titers and a statistical analysis, it appeared that the difference between the 3.0 µg dose groups (intradermal with a single hypodermic needle and intradermal with the Micronjet device) in respect of the B/Malaysia virus was significant, in that the Micronjet group had statistically significantly higher HAI titers (p=0.001) with respect to this B-type virus. From the same analysis it appeared that the difference between the 15 µg dose group (intramuscular) and the 3.0 µg Micronjet group in respect of the A/Wisconsin strain was also significantly different in that the group receiving the vaccine through the Micronjet device had statistically significant higher HAI titers (p=0.008) than the group receiving the five-times higher dose through intramuscular delivery.

All in all, it is concluded that an intradermal delivery of an influenza vaccine results in sufficient seroprotection- and seroconversion rates. Moreover, it can be concluded that such rates can be achieved even when much lower doses (3, 4.5 and 6 µg HA of each strain) are administered than generally used in influenza vaccine campaigns and set-ups (15 µg HA of each strain). Furthermore, it is concluded that it is preferred to use a multi-channel delivery device and/or at least a device that has a steady and standard injection-depth to achieve "real" intradermal delivery (without going beyond the dermis). Since no dose related increase could be detected in this study for instance such that a 3.0 µg HA dose performed less than a 4.5 µg or a 6.0 µg dose, it is likely that when an intradermal delivery is used with a virosome-based influenza vaccine such as the Inflexal^{®} vaccine, as disclosed herein, the dose may be lowered further. In other words, it may be concluded that a plateau is already reached when using 3.0 µg HA of each strain, at least when administered intradermally. Whether lower doses than 3.0 µg HA per strain can be used remains to be investigated.

Table I. Baseline characteristics of the 2^{nd} phase II clinical study using intradermal delivery of Inflexal^{®} influenza vaccine

| | IM 15 µg | ID | | | ID MicronJet 3 µg |
|---|---|---|---|---|---|
| | | 3 µg | 4.5 µg | 6 µg | |
| Number *n* | 56 | 56 | 56 | 56 | 56 |
| Female *n* | 30 | 31 | 24 | 30 | 34 |
| Age *mean* [y] | 36.1 | 39.5 | 38.4 | 39.6 | 34.1 |

| GMT pre-test *titer* | | | | | |
|---|---|---|---|---|---|
| A/Solomon Islands | 20.9 | 27.4 | 25.4 | 16.4 | 22.9 |
| A/Wisconsin | 40.9 | 52.9 | 45.3 | 34.0 | 38.3 |
| B/Malaysia | 8.5 | 10.5 | 9.0 | 8.1 | 6.4 |

### REFERENCES

Belshe R.B. et al. (2004). Serum antibody responses after intradermal vaccination against influenza. New Engl. J. Med. 351:2286-2294.
Glück R. (1992). Immunopotentiating reconstituted influenza virosomes (IRIVs) and other adjuvants for improved presentation of small antigens. Vaccine 10:915-920
Hosaka Y. et al. (1983). Hemolysis by liposomes containing influenza virus hemagglutinins. J. Virol. 46:1014-1017.
Huang R.T. et al. (1979). Association of the envelope glycoproteins of influenza virus with liposomes--a model study on viral envelope assembly. Virology 97:212-217.
Huckriede A. et al. (2005). The virosome concept for influenza vaccines. Vaccine 23 Suppl. 1:S26-38.
Kawasaki K. et al. (1983). Membrane fusion activity of reconstituted vesicles of influenza virus hemagglutinin glycoproteins. Biochim. Biophys. Acta. 733:286-290.
Kenney R.T. et al. (2004). Dose sparing with intradermal injection of influenza vaccine. New Engl. J. Med. 351:2295-2301.

## Claims

1. A virosomal preparation comprising influenza hemagglutinin (HA) antigen wherein the preparation does not contain an additional adjuvant, for use as an intradermal influenza vaccine in human subjects.

2. A preparation according to claim 1, comprising HA antigen from two or more influenza virus strains for use as an intradermal influenza vaccine in human subjects

3. A preparation according to any one of claims 1-2, wherein the concentration HA antigen from each influenza strain is between 1 and 10 µg per influenza strain, preferably about 3.0 µg per influenza strain.

4. A preparation according to any one of claims 1-3, wherein said preparation does not contain a trivalent combination of HA antigens from influenza strains A/New Caledonia/20/99, A/Moscow/10/99 and B/Hong Kong/330/2001.

5. Use of a virosomal preparation comprising influenza HA antigen in the manufacture of an influenza vaccine for intradermal administration in human subjects, wherein the preparation does not contain an additional adjuvant.

6. Use according to claim 5, wherein said preparation comprises HA antigen from two or more influenza virus strains.

7. Use according to any one of claims 5-6, wherein the concentration HA antigen from each influenza strain is between 1 and 10 µg per influenza strain, preferably about 3.0 µg per influenza strain.

8. Use according to any one of claims 5-7, wherein said vaccine is manufactured in a single dose volume of about 0.1 mL.

9. A kit comprising:
a) a virosomal preparation comprising influenza hemagglutinin (HA) antigen for vaccination of human subjects against influenza, wherein the preparation does not contain an additional adjuvant and wherein the preparation does not contain antigens from viruses other than influenza virus; and
b) a delivery device suitable for intradermal delivery of vaccines.

10. A kit according to claim 9, wherein said delivery device contains two or more separate delivery channels, such as microneedles or MicroPyramids, preferably said delivery device contains four or more separate delivery channels.

11. A kit according to claim 9 or 10, wherein the virosomal preparation comprises HA antigen from two or more influenza virus strains.

12. A kit according to any one of claims 9-11, wherein the concentration HA antigen from each influenza strain is between 1 and 10 µg per influenza strain, preferably about 3.0 µg per influenza strain.

13. A kit according to any one of claims 9-12, wherein said preparation does not contain a trivalent combination of HA antigens from influenza strains A/New Caledonia/20/99, A/Moscow/10/99 and B/Hong Kong/330/2001.

## Patentansprüche

1. Virosomales Präparat umfassend Influenza-Hämagglutinin (HA)-Antigen, wobei das Präparat kein zusätzliches Adjuvans enthält, zur Verwendung als intradermaler Influenzaimpfstoff beim Menschen.

2. Präparat zur Verwendung als intradermaler Influenzaimpfstoff beim Menschen nach Anspruch 1, umfassend HA-Antigen von zwei oder mehreren Influenzavirusstämmen.

3. Präparat zur Verwendung als intradermaler Influenzaimpfstoff beim Menschen nach einem der Ansprüche 1 bis 2, wobei die HA-Antigen-Konzentration von jedem Influenzastamm zwischen 1 und 10 µg pro Influenzastamm, bevorzugt etwa 3.0 µg pro Influenzastamm ist.

4. Präparat zur Verwendung als intradermaler Influenzaimpfstoff beim Menschen nach einem der Ansprüche 1 bis 3, wobei das Präparat keine trivalente Kombination von HA-Antigenen der Influenzastämme A/New Caledonia/20/99, A/Moscow/10/99 und B/Hong Kong/330/2001 enthält.

5. Verwendung eines virosomalen Präparats umfassend Influenza-HA-Antigen in der Herstellung eines Influenzaimpfstoffs zur intradermalen Verabreichung beim Menschen, wobei das Präparat kein zusätzliches Adjuvans enthält.

6. Verwendung nach Anspruch 5, wobei das Präparat HA-Antigen von zwei oder mehreren Influenzavirusstämmen umfasst.

7. Verwendung nach einem der Ansprüche 5 bis 6, wobei die HA-Antigen-Konzentration von jedem Influenzastamm zwischen 1 und 10 µg pro Influenzastamm, bevorzugt etwa 3.0 µg pro Influenzastamm ist.

8. Verwendung nach einem der Ansprüche 5 bis 7, wobei der Impfstoff in einem Einzeldosisvolumen von etwa 0.1 ml hergestellt ist.

9. Kit umfassend:
a) ein virosomales Präparat umfassend Influenza-Hämagglutinin (HA)-Antigen zur Impfung von Menschen gegen Influenza, wobei das Präparat kein zusätzliches Adjuvans enthält und wobei das Präparat keine Antigene von Viren außer Influenzaviren enthält; und
(b) eine Verabreichungsvorrichtung, die zur intradermalen Verabreichung von Impfstoffen geeignet ist.

10. Kit nach Anspruch 9, wobei die Verabreichungsvorrichtung zwei oder mehr getrennte Verabreichungskanäle, wie Mikronadeln oder MicroPyramids, enthält, wobei die Verabreichungsvorrichtung bevorzugt vier oder mehr getrennte Verabreichungskanäle enthält.

11. Kit nach Anspruch 9 oder 10, wobei das virosomale Präparat HA-Antigen von zwei oder mehr Influenzavirusstämmen umfasst.

12. Kit nach einem der Ansprüche 9 bis 11, wobei die HA-Antigen-Konzentration von jedem Influenzastamm zwischen 1 und 10 µg pro Influenzastamm, bevorzugt etwa 3.0 µg pro Influenzastamm ist.

13. Kit nach einem der Ansprüche 9 bis 12, wobei das Präparat keine trivalente Kombination von HA-Antigenen der Influenzastämme A/New Caledonia/20/99, A/Moscow/10/99 und B/Hong Kong/330/2001 enthält.

## Revendications

1. Préparation virosomale comprenant un antigène d'hémagglutinine grippale (HA) dans laquelle la préparation ne contient pas d'adjuvant supplémentaire, destinée à une utilisation en tant que vaccin intradermique contre la grippe chez les sujets humains.

2. Préparation destinée à une utilisation en tant que vaccin intradermique contre la grippe chez les sujets humains selon la revendication 1, comprenant un antigène HA provenant de deux souches grippales ou plus.

3. Préparation destinée à une utilisation en tant que vaccin intradermique contre la grippe chez les sujets humains selon l'une quelconque des revendications 1 à 2, dans laquelle la concentration en antigène HA de chaque souche grippale est comprise entre 1 et 10 µg par souche grippale, de préférence d'environ 3,0 µg par souche grippale.

4. Préparation destinée à une utilisation en tant que vaccin intradermique contre la grippe chez les sujets humains selon l'une quelconque des revendications 1 à 3, dans laquelle ladite préparation ne contient pas de combinaison trivalente d'antigènes HA des souches grippales A/New Caledonia/20/99, A/Moscow/10/99 et B/Hong Kong/330/2001.

5. Utilisation d'une préparation virosomale comprenant un antigène HA de la grippe dans la fabrication d'un vaccin contre la grippe pour une administration intradermique chez les sujets humains, dans laquelle la préparation ne contient pas d'adjuvant supplémentaire.

6. Utilisation selon la revendication 5, dans laquelle ladite préparation comprend de l'antigène HA provenant de deux souches du virus de la grippe ou plus.

7. Utilisation selon l'une quelconque des revendications 5 à 6, dans laquelle la concentration en antigène HA de chaque souche grippale est comprise entre 1 et 10 µg par souche grippale, de préférence d'environ 3,0 µg par souche grippale.

8. Utilisation selon l'une quelconque des revendications 5 à 7, dans laquelle ledit vaccin est fabriqué dans un volume monodose d'environ 0,1 ml.

9. Trousse comprenant :
a) une préparation virosomale comprenant un antigène d'hémagglutinine (HA) grippale destinée à la vaccination des sujets humains contre la grippe, dans laquelle la préparation ne contient pas d'adjuvant supplémentaire, et dans laquelle la préparation ne contient pas d'antigènes de virus autres que le virus de la grippe ; et
b) un dispositif de délivrance adapté pour une administration intradermique des vaccins.

10. Trousse selon la revendication 9, dans laquelle ledit dispositif de délivrance contient deux canaux de distribution distincts ou plus, tels que des micro-aiguilles ou des MicroPyramids, de préférence ledit dispositif de délivrance contient quatre canaux de distribution distincts ou plus.

11. Trousse selon la revendication 9 ou 10, dans laquelle la préparation virosomale comprend un antigène HA provenant de deux souches de virus de la grippe ou plus.

12. Trousse selon l'une quelconque des revendications 9 à 11, dans laquelle la concentration en antigène HA de chaque souche grippale est comprise entre 1 et 10 µg par souche grippale, de préférence d'environ 3,0 µg par souche grippale.

13. Trousse selon l'une quelconque des revendications 9 à 12, dans laquelle ladite préparation ne contient pas de combinaison trivalente d'antigènes HA des souches grippales A/New Caledonia/20/99, A/Moscow/10/99 et B/Hong Kong/330/2001.
